# EUROPEAN PATENT SPECIFICATION

(11) **EP 1 563 306 B1**
(45) Date of publication and mention of the grant of the patent: **14.02.2007**
(21) Application number: 04797261.7
(22) Date of filing: 23.11.2004
(51) Int. Cl.: G01N 33/543

(54) **PHOTOLINKER MACROMOLECULES, METALLIC SUBSTRATES AND LIGANDS MODIFIED WITH SAID LINKERS, AND PROCESS OF PREPARATION THEREOF**
PHOTOLINKER-MAKROMOLEKÜLE, MIT DEN LINKERN MODIFIZIERTE METALLISCHE SUBSTRATE UND LIGANDEN SOWIE VERFAHREN ZUR HERSTELLUNG DAVON
MACROMOLECULES POUR LA LIAISON PHOTOCHIMIQUE, SUBSTRATS METALLIQUES ET LIGANDS MODIFIES AVEC CES MACROMOLECULES ET PROCEDE POUR LEUR PREPARATION

(30) Priority: 28.11.2003 EP 03405851
(43) Date of publication of application: 17.08.2005
(73) Proprietor: C.S.E.M. CENTRE SUISSE D'ELECTRONIQUE ET DE MICROTECHNIQUE SA, 2007 Neuchâtel (CH)
(72) Inventor: SIGRIST, Hans, CH-3309 Kernenried (CH); CHAI GAO, Hui, CH-2000 Neuchâtel (CH); SOURY-LAVERGNE, Isabelle, CH-1000 Lausanne 26 (CH)
(74) Representative: Savatier, Yves
(86) International application number: PCT/CH2004/000704
(87) International publication number: WO 2005/052580

(56) References cited:
- WO-A-94/24561
- US-A- 5 242 828
- SIGRIST H ET AL: "SURFACE IMMOBILIZATION OF BIOMOLECULES BY LIGHT" OPTICAL ENGINEERING, SOC. OF PHOTO-OPTICAL INSTRUMENTATION ENGINEERS. BELLINGHAM, US, vol. 34, no. 8, 1 August 1995 (1995-08-01), pages 2339-2348, XP000518229 ISSN: 0091-3286
- CHEVOLOT YANN ET AL: "Synthesis and characterization of a photoactivatable glycoaryldiazirine for surface glycoengineering" BIOCONJUGATE CHEMISTRY, vol. 10, no. 2, March 1999 (1999-03), pages 169-175, XP002319912 ISSN: 1043-1802
- H. GAO, S. GUINCHARD, F. CREVOISIER, I. CAELEN, H. SIGIRIST: "Bioengineering of Metals with OptoDex® S" December 2003 (2003-12), ANNUAL REPORT 2003 OF CENTRE SUISSE D'ELECTRONIQUE ET MICROTECHNIQUE , NEUCHATEL; CH , XP002319913 Published in the Internet on 14.09.2004 under the address http://www.csem.ch/corporate/Report2003/pd f/p71.pdf page 71

## Description

The invention relates to a new photolinker macromolecule containing sulfur groups, which is attached to a metallic substrate by chemisorption or sulfur-metal complex formation, and optionally covalently bonded to a ligand, generally a biomolecule, in particular for use in Surface Plasmon Resonance biosensors, as well as a process for preparing same.

US Pat. Nos. 5,242,828; 5,436,161 and Lofas and Johnsson, 1990, J. Chem. Soc. Chem. Commun. 1526-28, disclose the chemical modification of metallic surfaces, such as gold surfaces, with the view to preparing sensing surfaces capable of selective molecular interactions for use in Surface Plasmon Resonance (SPR) biosensor systems. Those documents describe matrix coatings on metallic surfaces, in particular gold surfaces, suitable for use in biosensors. Free electron metal surface coating is achieved by attaching a densely packed monolayer of organic molecules having hydrocarbon chains, optionally interrupted by heteroatoms, of a length exceeding ten atoms with functions allowing the interaction with the free electron metal surface on one side, for instance asymmetrical or symmetrical disulfides, and an active group on the other side for covalently binding a biocompatible porous matrix via which a desired ligand can be bound. The matrix is a swellable organic polymer or a polysaccharide, for instance a carboxymethyldextran.

The sensing surfaces thus prepared have proved useful in biosensors using SPR on gold surfaces to monitor the molecular interaction between one binding-partner, referred to as the ligand or the probe, and the other binding-partner referred to as the analyte, without any labeling. However they have the following drawbacks:
- there is an intermediate hydrophobic layer (the monolayer of organic molecules) between the ligand and the metal, which may lead to unspecific binding of the analyte to the ligand,
- due to the superposition of two layers, viz. a hydrophobic monolayer covalently attached to a polymer matrix, there is a large distance of the ligand to the metallic surface, and therefore of the zone of molecular interaction between the ligand and the analyte to the metallic surface. This lowers the sensitivity (the evanescent field decreases exponentially with the distance to the metallic surface).

The problem addressed by the invention is to find means and methods for preparing biosensing surfaces, in particular for use in SPR biosensors, which do not have the above drawbacks.

EP 0484 472 describes the chemical modification of non-metallic surfaces to achieve desired chemical and physical surface characteristics and covalent binding of biomolecules thereto. Latent reactive groups are used to achieve covalent coupling of reagents such as biomolecules with derivatized biopolymers or synthetic polymers to various non-metallic substrates. The preferred latent reactive group is typically described as a photochemically reactive functional group, i.e. a photoactivable group. When exposed to an appropriate energy source, a photoactivable group undergoes a transition from an inactive state to a reactive intermediate capable of forming covalent bonds with appropriate materials or molecules (see e.g. Sigrist et al., 1995, Optical Engineering, Vol 34, No. 8, 2339-2347). Such linking agents, for example polysaccharide-based photolinker polymers that are substituted with photoreactive groups (see Caelen et al., 2002, Langmuir, Vol 18, 2463-2467 ), can be used for attaching proteins to silicon nitride and polystyrene.

The above problem is solved by the invention as defined in the appended claims.

Indeed, the invention provides a photolinker macromolecule and a method for preparing therewith sensing surfaces wherein there is no hydrophobic barrier between the ligand and the metallic surface and the distance of the ligand to the metallic surface is smaller than in the prior art, thus allowing more sensitivity in SPR biosensors.

That photolinker macromolecule can also be used for preparing microarrays, or for preparing metal-based and light-activatable nanoparticles, nanoassemblies and microparticles useful in the bioanalytics field, or the pharmaceutical or textile industry.

The invention concerns a new photolinker macromolecule, which is a saccharide-based polymer that contains photoactivable groups apt to be activated at a wavelength of at least 320 nm, and sulfur-containing groups, the sulfur-containing groups being selected from the group consisting of thiol (-SH), thioacid (-COSH), dithioacid (-CSSH), sulfide (-S-) and disulfide (-SS-), attached to a metallic substrate.

The term "photolinker" here means, as usual in the art (see e.g. Sigrist et al., 1995 and Caelen et al., 2002, references cited above), that the macromolecule is capable of linking a substrate to a ligand using a photoreaction.

The polysaccharide-based polymer, which contains photoactivable groups and sulfur-containing groups is generally derivatized from a polysaccharide by multiple substitutions with photoactivable groups and sulfur-containing groups.

The polysaccharide is suitably a linear or branched polysaccharide or an artificial polymer with polysaccharide substitutions, which has a molecular weight from 1000 to 5'000'000 dalton, preferably from 5'000 to 200'000 dalton, in particular from 10'000 to 70'000 dalton.

The polysaccharide may be for example a polysaccharide such as agarose, dextran, carrageenan, alginic acid, starch, and cellullose, or derivatives of these such as, e.g. carboxymethyl derivatives or amino derivatives.

Polysaccharides of the dextran type, which are non-crystalline in character, in contast to e.g. cellulose or agarose, are very suitable in those contexts. A preferred polysaccharide is thus dextran, in particular amino-dextran or carboxymethyl-dextran.

Following oxidative ring opening of polymer constituting monosaccharides at vicinal hydroxyl functions and introduction of amino groups at generated aldehydes the latter chemical functions become available for functionalization. The degree of substitution with sulfur-containing functional groups and photoactive groups depends on the extent of the primary oxidative cleavage of polysaccharide forming monosaccharides.

There are thus several ways of adjusting the final extent of total functional groups (sulfur-containing groups and photoactivable groups) in the polysaccharide-based polymer.

In one preferred procedure, the final degree of total substitution is set by the primary oxidative ring opening and the substitution with sulfur-containing groups and photoactivable groups is realized at a deliberate chosen ratio.

Alternatively, a variable degree of substitution is set by mixing a polysaccharide-based polymer with a high degree of substitution with a polysaccharide-based polymer having a low degree of substitution.

In another embodiment, the degree of substitution with the sulfur-containing groups and the photoactive groups are varied by varying the reaction conditions (e.g. reagent concentration, incubation time, or temperature) of the polymer functionalization reaction. The total available amino functions in amino dextran or carboxy functions in carboxymethyl dextran can vary between 0.005 to 1 mol per mol glucose monomer, a preferred range for subsequent functionalization with both the photoactivatable groups0 and the sulfur-containing groups being 0.01 to 0.5 mol per mol glucose monomer.

The sulfur-containing groups are groups capable of chemisorption to a metal. Such groups have been described for modification of gold surfaces by, for example, Nuzzo R.G. et al., 1983, Porter M. D. et al., 1987, and Troughton E.B. et al., 1988. Suitable sulfur-containing groups are thiol (-SH), thioacid (-COSH), dithioacid (-CSSH), sulfide (-S-) and disulfide (-SS-).

Reagents for introducing sulfur-containing groups into the polysaccharide-based polymer are (N-succinimidyl 3-(2-pyridyldithio)propionate, Sulfosuccinimidyl 6-(3'-(2-pyridyldithio)-propionamido) hexanoate, sulfosuccinimidyl-6 (alpha-methyl-alpha-(2-pyridyldithio)toluamido) hexanoate, N-succinimidyl S-acetylthioproprionate, 3-((2-aminoethyl)dithio)propionic acid, dithiobis(succinimidyl proprionate), dimethyl3,3'dithiobispropionimidate and others as provided by Fluka Chemicals, Molecular Probes, Pierce and other suppliers of special chemicals.

The photoactivable groups apt to be activated at a wavelength of at least 320 nm are groups that upon photoactivation at a wavelength of at least 320 nm generate intermediates apt to undergo insertion reactions with covalent bonds of biomolecules. Suitable photoactivable groups are derived from photoactivable reagents such as, for example aryldiazirines, which photogenerate carbenes, benzophenones, which photogenerate the ketyl radicals of the benzophenone moiety, and arylazides, which generate nitrenes.

Preferred photoactivable groups are aryldiazirines and benzophenones. Aryldiazirines and benzophenone based photoactive groups are photochemically activated and the photochemical intermediates are generated by irradiation with actinic light at 350 nm. Any light source is applicable presuming sufficient irradiance at the wavelength requested. A specific requirement for the photoactivation of linker polymers bound to metallic substrates is the elimination of high energy radiation by filtering all incident light below 320 nm with a cut-off filter, such as not to break the chemisorptive linkage between the metal and the sulfur-containing group. Preferred activation of sulfur-containing photolinker polymers on metal surfaces is effected, for instance, by irradiation for 4 min with, for instance an Oriel Lamp (350 nm, 11 mW/cm²). Alternative light sources that generate the same irradiance can be used instead (e.g. Stratalinker with 365 nm tubes, 1 mw/cm², 45 min irradiation).

Examples of preferred reagents for introducing photoactivable groups are 4-(p-azidosalicylamido)butylamine, N-hydroxysuccinimidyl-4-azidosalicylic acid, p-azidophenyl-isothiocyanate, benzophenone-4-isothiocyanate, benzophenone-4-maleimide, 4-benzylbenzoic adic succimidyl ester, 3-(trifluoromethyl)-3-(m-isothiocyanophenyl) diazirine and other aryldiazirine containing reagents.

Suitable metals for the metallic substrate are free electron metals which show affinity for sulfur, such as e.g. copper, aluminum, cadmium, iron, manganese, silver, gold and platinum. Preferred metals are copper, aluminum, silver, gold, palladium and platinum.

The metallic substrate may widely vary in shape, e.g. plate-shaped, spheroid, cylindrical, pyramidal, or rod-like, and in size (from a few nanometers to any dimension). For use as a sensing surface in a SPR biosensor, the metallic substrate is generally plate-shaped.

The attachment to the metal occurs spontaneously at room temperature by chemisorption as described in the art for thioalkane self-assembled monolayers (see e.g. G.M. Whitesides, 1996, Ann. Rev. Biophys. Biomol. Struct., 25, 55-78), or by sulfur-metal complex formation processes. Incubation at elevated temperatures, from 30 to 60 °C, completes the reaction within a short time and stabilizes the interaction.

The invention also relates to the above photolinker macromolecule attached to a metallic substrate, which is covalently bonded to a ligand.

The ligand (also called "probe") can be any molecule of interest depending on the application. Generally it is a biomolecule, e.g. a protein or a nucleic acid, in an active form.

Covalent binding of the ligand to the photolinker macromolecule attached to a metallic substrate is preferably effected by submitting a mixture of those two components to a photoreaction. The photoactivable groups generate highly reactive species such as carbenes or nitrenes with very short half-times, which react with neighboring ligand molecules, forming covalent bonds. When the ligand is a biologically active biomolecule, the above photoreaction allows to retain its activity.

Covalent binding of the ligand to the photolinker macromolecule attached to a metallic substrate may also be effected thermally at elevated temperature.

The above photolinker macromolecule attached to a metallic substrate and covalently bonded to a ligand can be used as part of a biosensing surface of a biosensor system, particularly a SPR biosensor. Such a biosensor has an enhanced sensitivity thanks to the short distance between the ligand and the metallic surface. It can be used for all kinds of analytes, even those partially hydrophobic of low molecular weight, thanks to the absence of a hydrophobic barrier between the ligand and the metallic surface.

That macromolecule attached to a metallic substrate and covalently bonded to a ligand is also very useful as part of a microarray where a large number of different immobilized biomolecules such as proteins or nucleic acids are displayed.

That macromolecule attached to a metallic substrate and covalently bonded to a ligand may also be used for preparing, i.e. functionalizing, nanoparticles, nanoassemblies or microparticles. The latter may carry as ligand a biologically active substance, e.g. an antibacterial or a cosmetically or pharmaceutically active substance. Such particles or assemblies can be used in the healthcare industry, e.g. as means of administration of active substances, or in the textile industry, e.g. for immobilizing antibacterials on textile fibers.

Nano- or microparticles thus prepared are also of interest in the bioanalytics field.

The invention will be further described by the following examples which have an illustrative but not limitative character.

The description below will be better understood by referring to Figures 1, 2A, 2B, 2C, 2D, 3 and 4.
Figure 1 is a reaction scheme showing derivatization of Optodex A into Optodex S and Optodex SH.
Figure 2A is a photograph of a mask used for photopatterning comprising a lattice-shaped pattern of transparent (white) zones and light-absorbing (dark) zones.
Figure 2B is a fluorescence scanograph of an Optodex S treated gold plated platform to which Cy5-labeled riboflavin binding protein (RBP) has been added, after ultraviolet irradiation using the photomask of Figure 2A.
Figure 2C is a fluorescence scanograph of an Optodex S treated gold plated platform to which Cy3-labeled bovine serum albumin (BSA) has been added, after ultraviolet irradiation using the photomask of Figure 2A.
Figure 2D is a fluorescence scanograph of an Optodex S treated gold plated platform to which mouse immunoglobulin (m-IgG) has been added, after ultraviolet irradiation using the photomask of Figure 2A and immunocomplexation with Cy-5 labeled goat anti-mouse igG antibody.
Figure 3 is a fluorescence scanograph of 4 printed microarrays (arrays 1, 2, 3 and 4, each 20 x 20 spots, parallel printed with 4 pins) of fluorophore labeled proteins after photoimmobilization on a gold platform.
Figure 4 is a Surface Plasmon Resonance sensogram of vitamin B₂ binding to riboflavin binding protein (RBP) after its photoimmobilization on OptoDex S modified gold platform.

Optodex is a trade name for aryldiazirine functionalized dextran derivatives photolinker polymers described by Gao et al, 2003, 57, No.10.

### EXAMPLE 1 Synthesis and characterization of OptoDex S and Optodex SH

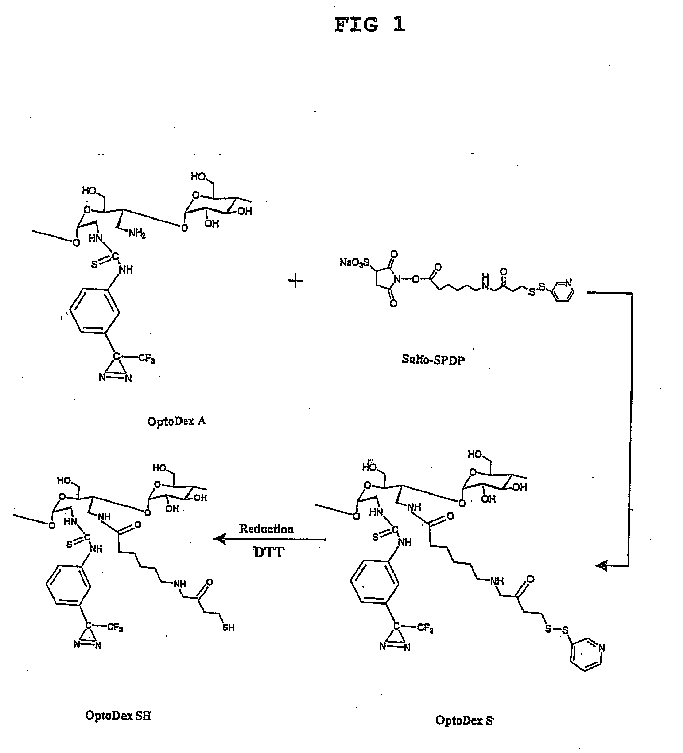

### a) Synthesis of Optodex S and Optodex S-H (cf. Figure 1)

Each of OptoDex S and OptoDex SH (reduced OptoDex S) is a photolinker polymer which is a multiply substituted aminodextran functionalized with both photoactive and sulfur (thiol and/or disulfide) containing substituents. The synthesis of those photolinker polymers is illustrated in Figure 1.

The starting material is photolinker polymer Optodex A, which is obtained by partial thiocarbamoylation of aminodextran with photolinking reagent 3-(trifluoromethyl)-3-(m-isothiocyanophenyl) diazirine (see I. Caelen, H. Gao and H. Sigrist, Langmuir, Vol 18, 2463-2467). That photolinker polymer is derivatized on the residual amino functions with the reagent sulfosuccinimidyl-6-[3'-(2-pyrimidylditihio)-propionamido] hexaoate (LC sulfo SPDP). The reaction product is photolinker polymer OptoDex S carrying an activated disulfide group. Treatment of OptoDex S with the reducing agent dithiothreitol yields a reduced form thereof carrying free thiol functions, photolinker polymer OptoDex SH.

OptoDex S is prepared according to the following detailed procedure. Aminodextran of molecular weight 40'000 dalton, available from Molecular Probes, is derivatized with the photoactive reagent 3-(trifluoromethyl)-3-(m-isothiocyanophenyl) diazirine by reaction of aminodextran at 2.0 mg/ml and the diazirine aryl isothiocyanate at 0.3 mg/ml in 0.1 M sodium carbonate buffer, pH 9.0 for 5hours at 37°C. Upon completion of the reaction, excess photoreagent is removed by exclusion chromatography on Sephadex G 25 in diluted aqueous buffer (1:100 diluted phosphate buffered saline). Fractions eluting in the void volume contain the substituted aminodextran product: OptoDex A ,functionalized with the above photoreactive reagent. Pooled fractions are lyophilized and stored at -18°C, as required. For further functionalization, OptoDex A containing approximately 14 mol amino groups per mol OptoDex A, is dissolved in phosphate buffered saline (PBS), pH 7.4 at a concentration of 2.0 mg/ml. To this solution, 100 µl of a freshly prepared solution of LC-Sulfo-SPDP (5.2 mg/ml phosphate buffered saline) is added. The mixture of the two solutions is incubated at ambient temperature with continuous stirring. Upon completion of the reaction (after about 1 h) the reaction mixture is purified by chromatography on a PD 10 column (Pharmacia) using phosphate buffered saline containing 1 mM EDTA pH 7.4 as elution buffer. Fractions of 1 ml are collected and the absorption at 350 nm is recorded for product identification. Unless immediately used, the product, OptoDex S, is stored at - 18 °C.

OptoDex S contains activated disulfide groups allowing facilitated disulfide exchange reactions. The reduced form of OptoDex S is obtained by treatment of OptoDex S with 5 mM dithiothreitol in 10 mM sodium acetate buffer, pH 5.0 and incubation of the reaction mixture at 37 °C for 60 minutes. The reaction product is OptoDex SH (containing free thiol groups). It is purified by chromatography on Sephadex G10. Immediate use of the product is recommended as inter- and intramolecular disulfide bridge formation may spontaneously occur.

### b) Characterization of OptoDex S

The product OptoDex S is supposed to contain photoactive aryldiazirine functions as well as activated disulfide functions. The presence of each of those chemical functions is demonstrated by the two analytical procedures outlined below. In each of those procedures, a sample of OptoDex A (photoactive, but not containing disulfides or thiols) serves as control and an additional analytical control includes samples which are not exposed to activating light.

### Determination of the sulfide content of OptoDex S.

The sulfide content of Optodex S is determined by reducing Otodex S to OptoDex SH as described above and determining the content of free thiols thereof using the thiol and sulfide quantitation kit T-6060, available from Pierce Chemicals. OptoDex S is dissolved at a concentration of 0.2 mg/ml in deionized water and 100 µl of the solution is pipeted into a microplate (Nunc-Immuno Module Polysorp F8). The microplate material is polystyrene and 8 replicas of each sample are prepared with OptoDex S as well as with OptoDex A serving as control. After adsorptive binding for 90 minutes at ambient temperature the microplate wells are rinsed with water and dried in vacuum for 90 minutes at 5x10⁻² mbar. Coated wells are then irradiated for 4 minutes with the Oriel light source (wavelength 350 nm, irradiance 11 mW/cm²) and subsequently rinsed with phosphate buffered saline containing 0.05% Tween (3 times), followed by phosphate buffered saline (3 times) and deionized water (3 times). Control samples (- Light) are identically treated but not irradiated. For sulfide quantitation purposes, photoimmobilized OptoDex S (or OptoDex A as control) is treated with 100 µl 5mM dithiothreitol in 10 mM sodium acetate buffer, pH 5.0, yielding Optodex SH. All samples are incubated during 60 minutes at 37 °C and then rinsed 3 times with deionized water. The presence thiols generated from OptoDex SH is analyzed with the thiol and sulfide quantitation kit (colorimetric assay). The results are summarized in the table below.

| Samples | Thiol groups (pmol / well) | |
|---|---|---|
| | + Light activated | - Light |
| OptoDex S | 29.8 ± 4.9 | 4.2 ± 2.3 |
| OptoDex A | 5.5 ± 0.40 | 2.3 ± 2.2 |

The above table shows that photoimmobilized OptoDex SH contains approximately 20 pmol reactive thiol SH groups (equivalent to 10 pmol disulfide group for OptoDex S) per well, corresponding to about 500 fmol per mm² polystyrene.

### Photoactivity of OptoDex S.

The photoactivity of OptoDex S is tested by light-dependent immobilization of the enzyme alkaline phosphatase onto polystyrene, using as photolinker polymer OptoDex S (or OptoDex A as control).

OptoDex S is mixed with alkaline phosphatase (2000-3000 DEA units per mg protein, purchased from Sigma) in a weight ratio 8:1 (w/w), and applied to micro plate wells (Nunc-Immuno Module Polysorp F8, from Nunc, Denmark). The final quantity of alkaline phosphatase added to each well is 200 ng. The volume added per well is 40 µl, adjusted with diluted (1:100) phosphate buffered saline containing 10% ethanol (v/v). After drying for 3 hours under vacuum (1 hour at 20 mbar, 2 hours at 5 x 10⁻² mbar), coated surfaces are irradiated for 4 minutes with the Oriel Lamp (wavelength 350 nm, 11 mW/mm²). The surfaces are then rinsed with PBS/Tween^{™} (3 times), PBS (3 times) and deionized water (3 times). Each rinsing step consists of shaking for 5 minutes. Eight replicates of each sample were tested.

The enzymatic activity of photoimmobilized alkaline phosphatase is detected using the Alkaline Phosphatase Substrate Kit from Pierce. The substrate solution is freshly prepared according to the product description and applied to wells (100 µl/well). Microplates are incubated during 30 minutes at 37°C, and the reaction is stopped by addition of NaOH, 2.0N (50 µl/well). Color developed is determined by measuring the absorption at 405 nm with a commercial microplate reader. The results obtained with 8 replicate samples are summarized in the table below (+ Light: exposure of the sample to activating light; -Light: no exposure to activating light).

| | Surface content | Assay condition | Alk. Phosphatase activity ( E 450 nm) |
|---|---|---|---|
| Sample | OptoDex S and alkaline phosphatase | + Light | 2.333 |
| | | - Light | 0.058 |
| Control 1 | OptoDex A and alkaline phosphatase | + Light | 2.256 |
| | | - Light | 0.064 |
| Control 2 | OptoDex S | + Light | 0.055 |

The above table shows that upon light activation OptoDex S, like Optodex A, immobilizes alkaline phosphatase on polystyrene while retaining its activity.

### EXAMPLE 2 Coating of gold surfaces with OptoDex S and mask-assisted study of photoimmobilization thereon of biomolecules

The gold platforms used for OptoDex S coating are silicon chips of 12 x 12 mm with a top 150 nm gold layer on a Si₃N₄ coated silicon wafer. Those chips are first rinsed 3 times with isopropanol and cleaned with oxygen plasma for 3 minutes at room temperature.

### a) OptoDex S chemisorption on gold surfaces

OptoDex S coating of gold is achieved by adding to each gold coated platform 50 µl of a solution of 1.0 mg OptoDex S per ml diluted (1:100) phosphate buffered saline to the gold coated chip. To achieve homogeneous chemisorption of OptoDex S, the platforms are kept in a humid chamber for 5 hours at 37 °C. After this step, platforms are rinsed with deionized water and dried for 1 hour at 5 x 10⁻² mbar. Unless immediately processed, OptoDex S treated gold surfaces are stored vacuum packed at -18 °C.

### b) Photoimmobilization of biomolecules to OptoDex S treated gold surfaces

Photoimmobilization of a biomolecule to OptoDex S treated gold surfaces is attained by diluting the biomolecule to a final concentration of 0.2 mg/ml with phosphate buffered saline (1:100). The biomolecules used in this set of experiments are fluorophore (Cy5)-labeled riboflavin binding protein, Cy3-labelled bovine serum albumin or non-labeled mouse immunoglobulin, To each Optodex S treated gold coated platform, 25 µl of the above described protein solution is added. Coated platforms are then dried in vacuum for 1.5 hour (30 minutes at 20 mbar followed by 1 hour at 5x10⁻² mbar). Covalent binding of the biomolecules to OptoDex S coated gold surfaces is achieved by irradiating the surfaces during 5 minutes with the Oriel light source (350 nm, irradiance 11 mW/cm², in presence of a UV cut-off filter), using a patterned photomask (see Figure 1A) for easy demonstration of the light-dependency of the immobilization reaction.

Following photoimmobilization the surface treated gold platforms are thoroughly rinsed by sequential treatment (5 minutes each) under continuous agitation with the following solutions: 1% serum albumin in phosphate buffered saline (once); phosphate buffered saline containing 0.02 % Tween^{™} 20 (3 times); phosphate buffered saline (3 times); deionized water (3 times).

Mouse immunoglobulin m-lgG, which carries no fluorophore is immunocomplexed with Cy-5 labeled goat anti-mouse igG antibody. Immunostaining with Cy5 labeled goat anti-mouse immunoglobulin antibody is attained by incubation of the platform during 30 minutes at 37 °C in a solution containing a diluted antibody preparation (1:200 diluted with phosphate buffered saline containing 1% bovine serum albumin). Prior to imaging, the immunostained surfaces are rinsed (3 times each) with phosphate buffered saline containing 0.05 % Tween 20, phosphate buffered saline and deionized water

Photopatterned fluorophore-labeled proteins are imaged by scanning with a confocal array scanner. The scanner settings on the Affymetrix Array scanner 428 being set at: gain 50, focus 0.1 for both Cy5 and Cy3.

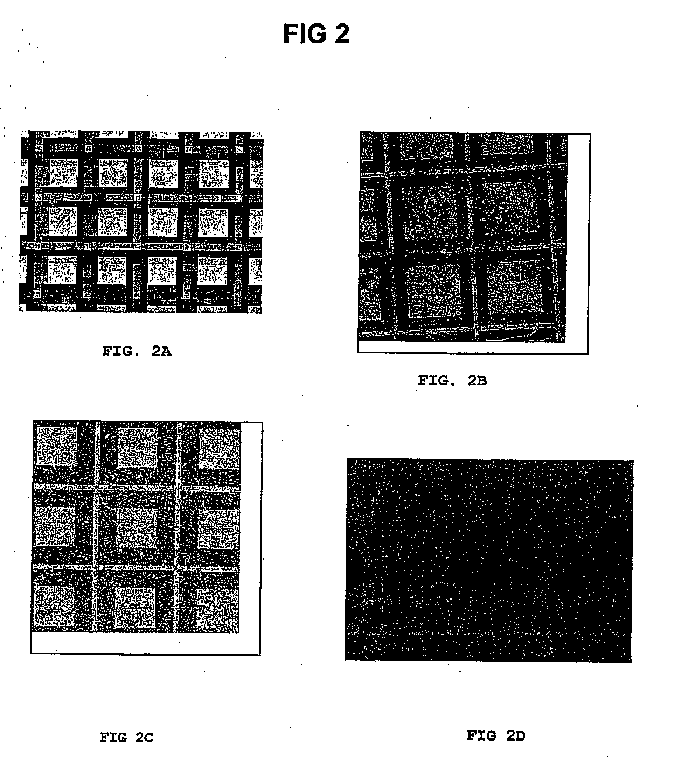

The results of fluorescence scanning are shown on the scanographs of Figures 2B, 2C and 2D.

Figure 2B is a fluorescence scanograph of an Optodex S treated gold plated platform to which Cy5-labeled riboflavin binding protein (RBP) has been added, after ultraviolet irradiation using the photomask of Figure 2A.

Figure 2C is a fluorescence scanograph of an Optodex S treated gold plated platform to which Cy3-labeled bovine serum albumin (BSA) has been added, after ultraviolet irradiation using the photomask of Figure 2A.

Figure 2D is a fluorescence scanograph of an Optodex S treated gold plated platform to which mouse immunoglobulin (m-IgG) has been added, after ultraviolet irradiation using the photomask of Figure 2A and immunocomplexation with Cy-5 labeled goat anti-mouse igG antibody.

Those scanographs show that each of the 3 biomolecules is immobilized on the OptoDex treated gold surface only in the zones of irradiation (appearing as light emitting) and not the zones where irradiation has been stopped by the mask.

### EXAMPLE 3 Coating of gold surfaces with Optodex S and study of photoimmobilization of biomolecules using standard microarray printing technology

This example demonstrates the use of OptoDex modified gold platforms for the manufacturing of microarrays with a conventional microarrayer (Affymetrix Array Printer 417). Gold platforms used in this set of experiments are commercial gold coated glass slides (75mm x 25mm x 1 mm) as available for instance from Erie SA (BioGold). Gold-coated slides are treated for 3 minutes with oxygen plasma to effect cleaning of the gold surface. The gold surface is chemisorbed with OptoDex S by incubating the slide platforms during 5 hours at 37°C in an OptoDex S solution (0.5 mg OptoDex S per ml diluted (1:100) phosphate buffered saline). After this treatment the slide surfaces are rinsed with deionised water and dried in a vacuum chamber during 1 hour at 5 x 10⁻² mbar. Such treated surfaces can be stored at -18°C until further use.

For demonstration purposes, two types of biomolecules are used for array printing: Cy5-labelled mouse immunoglobulin and Cy3 labeled bovine serum albumin. 400 spot repeat arrays of each biomolecule preparation are printed with the Affymetrix Array printer 427 (pin-and-ring printer, simultaneous printing with 4 pins). Covalent binding of the spotted biomolecules to OptoDex S coated gold surfaces is achieved by irradiating the surfaces during 5 minutes with the Oriel light source (350 nm, irradiance 11 mW/cm², with an UV cut-off filter). Following light exposure, the platforms are thoroughly rinsed by sequential treatment (5 minutes each) under continuous agitation with the following solutions: 1% serum albumin in phosphate buffered saline (once); phosphate buffered saline containing 0.02 % Tween^{™} 20 (3 times); phosphate buffered saline (3 times); deionized water (3 times). Printed arrays are imaged by reading with the Affymetrix Array reader.

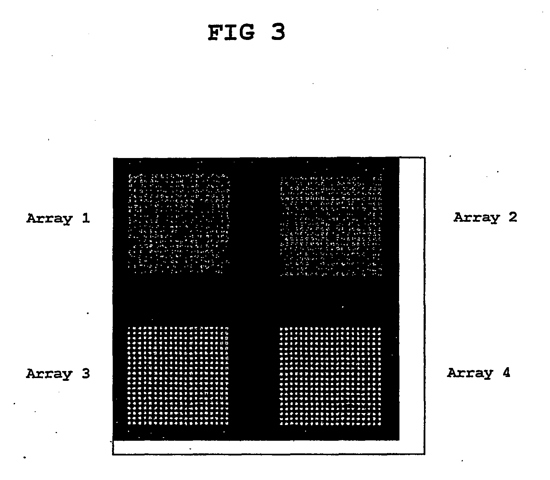

The result of such an experiment is shown in Figure 3. 400-spot microarrays are printed in parallel with the 4 pins of the Affymetrix Array Printer. Array 1 is a repeat array of a solution containing Cy5-labelled mouse immunoglobulin. Array 2 represents a repeat array of a solution containing a 1:1 mixture of Cy5-labelled mouse immunoglobulin and OptoDex A. Correspondingly, array 3 is a repeat array of a solution of Cy3-labelled bovine serum albumin. Array 4 shows a print of a solution containing a 1:4 mixture of Cy3 -labeled bovine serum albumin and OptoDex A.

This example shows the feasibility of using standard printing technology for making microarrays with a large number of different proteins immobilized thereon.

### EXAMPLE 4 Direct measurement of Vitamin B₂ binding to photoimmobilized riboflavin binding protein by Surface Plasmon Resonance on gold platforms

This sequence of experiments provides evidence that the riboflavin (i.e. vitamin B₂) binding protein RPB can be photoimmobilized on gold platforms. Furthermore, the conditions for RBP binding and assay conditions are elaborated with RBP photoimmobilized on microplates. Finally, evidence is provided that the combination of photoimmobilized RBP on gold can be used for the direct quantitative measurement of vitamin B₂.

The retention of biological activity of photoimmobilized RBP has been confirmed in a microplate assay system prior to elaboration of the vitamin B₂ binding assay on gold platforms. Two procedures for biomolecule immobilization have been tested: a) layer coating of RBP and b) co-immobilization of RBP, both systems use OptoDex A as linker polymer, and vitamin B₂ binding is detected by fluorescence quenching: Riboflavin (vitamin B₂) fluoresces in solution. The intrinsic fluorescence is quenched when the vitamin binds to RBP. Therefore, the more vitamin B₂ binds to RBP the stronger is the quenching of the intrinsic fluorescence of vitamin B₂. Fluorescence quenching is thus indicative for the bioactivity of the immobilized RBP. Both types of assay are carried out on polystyrene microplates. Out of the two procedures mentioned, co-immobilization yielded high vitamin B₂ binding activity and low unspecific binding. Based on the results obtained, the co-immobilization process is preferably used for probe molecule immobilization onto gold surfaces, as demonstrated with RBP.

### Photoimmobilization of riboflavin binding protein (RBP) on gold.

The experimental procedure for riboflavin binding protein (RBP) immobilization on gold surfaces is detailed in Example 2. Identical procedures are followed, with the difference however that native (non-labeled) RBP was used instead of fluorophore-labeled RBP.

### a) Pretreatment of gold platforms and functionalization with OptoDex S.

Gold platforms (Biacore Au Sensor chip) designed for the use in the Biacore QC analytical detection system are obtained from Biacore AB, Sweden. The gold surfaces are pretreated with oxygen plasma for 3 minutes at ambient temperature. OptoDex S is dissolved in phosphate buffered saline (1:100 diluted) at a final concentration of 1 mg/ml, and 50 µl of this solution is applied to the gold platform with a pipette. Binding of OptoDex S to gold is attained by incubation at 37°C for 5 hours at 80% humidity. Treated surfaces are then rinsed with deionized water and dried in a vacuum chamber during 1 hour at 5 x 10⁻² mbar. Resulting OptoDex S modified gold platforms are vacuum-packed and stored at -18°C until further used.

### b) Covalent attachment of RBP to OptoDex S modified gold platforms

RBP is dissolved in phosphate buffered saline (1:100 diluted) at a concentration of 0.2 mg/ml and applied to OptoDex S modified gold platforms platform (25 (µl/chip). Following layer coating procedures, RBP is mixed with OptoDex A (ratio1:2, w/w) in diluted (1:100) phosphate buffered saline and the mixture is applied to OptoDex S modified gold platforms. The platforms are then dried in vacuum during 30 minutes at 20 mbar and 1 hour at 5 x 10⁻² mbar. After drying, the platforms are irradiated for 5 minutes with Oriel Lamp (350 nm, 11 mW/cm²) in the presence of a UV cut-off filter to eliminate light of wavelengths shorter than 300 nm. After the attachment of RBP, the platforms are rinsed by applying the following treatment in this sequence: a) phosphate buffered saline containing 0.02 % Tween 20, b) phosphate buffered saline, c) deionized water, each rinsing step being carried out for 5 minutes under agitation. Platforms with covalently attached RPB are vacuum-packed and stored at -18°C until further used.

### c) Vitamin B₂ binding to RBP detected by Surface Plasmon Resonance.

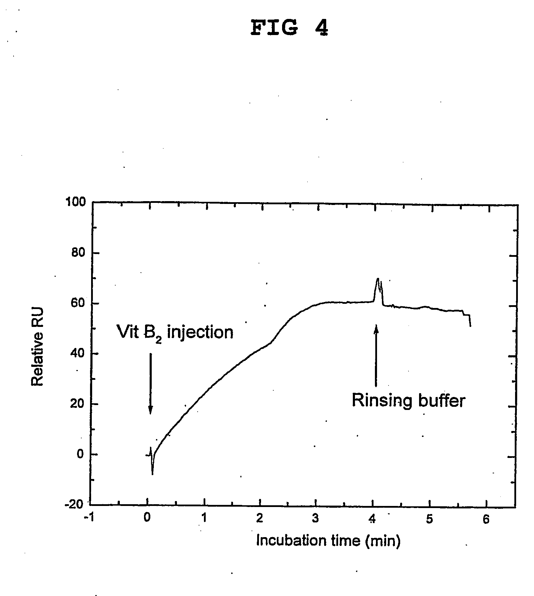

Detection of vitamin B₂ binding to photoimmobilized RBP is effected with the Biacore QC instrument. Biacore Au Sensor chips are modified as described above and introduced in the Bioacore QC instrument. A solution vitamin B₂ (5 to 50 µg vitamin B₂ in water containing 0.12 % v/v acetic acid) is injected and vitamin B₂ binding is registered by recording the mass change deposited on the surface (RU = relative units). In the experiment shown in Figure 4, saturation of vitamin B₂ binding is achieved in 4 minutes. After 4 minutes the surface is rinsed with deionized water containing 0.12 % acetic acid (rinsing buffer). The interaction of vitamin B₂ with the immobilized RBP can be reversed by exposure of the surface with a regeneration buffer consisting of 0.033 % sodium dodecyl sulfate in 0.1 M phosphate buffer, pH 4.5.

The above shows that biomolecules immobilized on a coating of OptoDex S on a gold surface retain their biological activity and therefore can act as ligands in a biosensor.

## Claims

1. Photolinker macromolecule, which is a saccharide-based polymer that contains photoactivable groups apt to be activated at a wavelength of at least 320 nm, and sulfur-containing groups, the sulfur-containing groups being selected from the group consisting of thiol (-SH), thioacid (-COSH), dithioacid (-CSSH), sulfide (-S-) and disulfide (-SS-), attached to a metallic substrate.

2. Macromolecule of claim 1, covalently bonded to a biomolecule in an active form.

3. Macromolecule of claim 1 or 2, wherein the polysaccharide is selected from the group consisting of agarose, dextran, carrageenan, alginic acid, starch, and cellullose, and a derivative thereof.

4. Macromolecule of any of any of claims 1 or 2, wherein the polysaccharide is dextran, in particular amino-dextran or carboxymethyl-dextran.

5. Macromolecule of claim 4, wherein the saccharide is amino-dextrane or carboxydextrane, the total amino functions or carboxy functions available for subsequent functionalization with both the photoactivatable groups and the sulfur-containing groups being 0.01 to 0.5 mol per mol glucose monomer.

6. Macromolecule of any of any of the preceding claims, wherein the photoactivable groups are selected from the group consisting of aryldiazirines and benzophenones.

7. Macromolecule of any of the preceding claims, wherein the photoactivable groups are selected from the group consisting 4-(p-azidosalicylamido)butylamine, N-hydroxysuccinimidyl-4-azidosalicylic acid, p o-phenone-4-maleimide, 4-benzylbenzoic adic succimidyl ester, or -azidophenyl-isothiocyanate, benzophenone-4-isothiocyanate, benz 3-(trifluoromethyl)-3-(m-isothiocyanophenyl) diazirine.

8. Macromolecule of any of the preceding claims, wherein the metal is selected from the group of aluminum, copper, gold, palladium, platinum and silver.

9. Sensing surface of biosensor, which comprises a macromolecule of claim 2.

10. Microarray, which comprises a macromolecule of claim 2.

11. Nanoparticle, nanoassembly or microparticle comprising a macromolecule of claim 2.

12. Method of preparing a photolinker macromolecule of claim 1 which comprises derivatizing a polysaccharide by multiple substitution with photoactivable groups and sulfur-containing groups, and attaching the derivatized saccharide to a metal by chemisorption or sulfur-metal complex formation processes.

13. Method of preparing a macromolecule of claim 2 which comprises submitting a mixture of a photolinker macromolecule of claim 1 and a biomolecule in an active form to a photoreaction at wavelength of at least 320 nm, in the absence of any incident light below 320 nm.

## Patentansprüche

1. An ein Metallsubstrat angeheftetes Photolinker-Makromolekül, das ein auf Saccharid beruhendes Polymer ist, das photoaktivierbare Gruppen, die bei einer Wellenlänge von mindestens 320 nm aktiviert werden können, sowie schwefelhaltige Gruppen enthält, wobei die schwefelhaltigen Gruppen aus der Gruppe von Thiol (-SH), Thiosäure (-COSH), Dithiosäure (-CSSH), Sulfid (-S-) und Disulfid (-S-S-) ausgewählt werden.

2. Makromolekül nach Anspruch 1, kovalent an ein Biomolekül in einer aktiven Form gebunden.

3. Makromolekül nach Anspruch 1 oder 2, worin das Polysaccharid aus der Gruppe von Agarose, Dextran, Carrageenan, Alginsäure, Stärke und Cellulose sowie einem Abkömmling davon ausgewählt ist.

4. Makromolekül nach einem der Ansprüche 1 oder 2, worin das Polysaccharid Dextran und insbesondere Aminodextran oder Carboxymethyldextran ist.

5. Makromolekül nach Anspruch 4, worin das Saccharid Aminodextran oder Carboxymethyldextran ist, wobei die Gesamtmenge der Aminofunktionen oder Carboxyfunktionen, die für eine nachfolgende Funktionalisierung sowohl mit den photoaktivierbaren Gruppen als auch mit den schwefelhaltigen Gruppen zur Verfügung stehen, 0,01 bis 0,5 Mol je Mol des Glucosemonomers darstellen.

6. Makromolekül nach einem der vorangehenden Ansprüche, worin die photoaktivierbaren Gruppen aus der Gruppe von Aryldiazirinen und Benzophenonen ausgewählt sind.

7. Makromolekül nach einem der vorangehenden Ansprüche, worin die photoaktivierbaren Gruppen aus der Gruppe von 4-(*p*-Azidosalicylamido)butylamin, N-Hydroxysuccinimidyl-4-azidosalicylsäure, *p*-Azidophenyl-isothiocyanat, Benzophenon-4-isothiocyanat, Benzophenon-4-maleimid, 4-Benzylbenzoesäure-Succimidylester oder 3-(Trifluormethyl)-3-(m-isothiocyanophenyl)diazirin ausgewählt werden.

8. Makromolekül nach einem der vorangehenden Ansprüche, worin das Metall aus der Gruppe von Aluminium, Kupfer, Gold, Palladium, Platin und Silber ausgewählt ist.

9. Sensoroberfläche eines Biosensors, die ein Makromolekül nach Anspruch 2 umfasst.

10. Mikroarray, der ein Makromolekül nach Anspruch 2 umfasst.

11. Nanopartikel, Nanogruppierung oder Mikropartikel, ein Makromolekül nach Anspruch 2 umfassend.

12. Verfahren zur Herstellung eines Photolinker-Makromoleküls nach Anspruch 1, das umfasst, ein Polysaccharid durch mehrfache Substitution mit photoaktivierbaren Gruppen und schwefelhaltigen Gruppen zu derivatisieren und das derivatisierte Saccharid durch Chemisorption oder Schwefel-Metall-Komplexbildungsprozesse an ein Metall anzufügen.

13. Verfahren zur Herstellung eines Makromoleküls nach Anspruch 2, das umfasst, ein Gemisch eines Photolinker-Makromoleküls nach Anspruch 1 und eines Biomoleküls in einer aktiven Form in Abwesenheit von jeglichem Lichteinfall unterhalb von 320 nm einer Photoreaktion bei einer Wellenlänge von mindestens 320 nm zu unterwerfen.

## Revendications

1. Macromolécule photolinker, qui est un polymère à base de saccharide qui contient des groupes photoactivables susceptibles d'être actives à une longueur d'onde d'au moins 320 nm, et des groupes contenant du soufre, les groupes contenant du soufre étant choisis dans le groupe constitué d'un thiol (-SH), un thioacide (-COSH), un dithioacide (-CSSH), un sulfure (-S-) et un disulfure (-SS-), attaché à un substrat métalique.

2. Macromolécule selon la revendication 1, liée de façon covalente à une biomolécule sous forme active.

3. Macromolécule selon la revendication 1 ou 2, dans laquelle le polysaccharide est choisi dans le groupe constitué de l'agarose, du dextrane, de la carraghénine, de l'acide alginique, de l'amidon, et de la cellulose, et d'un dérivé de l'un de ceux-ci.

4. Macromolécule selon la revendication 1 ou 2, dans laquelle le polysaccharide est du dextrane, en particulier de l'amino-dextrane ou du carboxyméthyl-dextrane.

5. Macromolécule selon la revendication 4, dans laquelle le saccharide est de l'amino-dextrane or du carboxydextrane, les fonctions totales amino ou carboxy disponibles pour une fonctionnalisation ultérieure avec à la fois les groupes photoactivables et les groupes contenant du soufre étant de 0,01 to 0,5 mol par mol de monomère glucose.

6. Macromolécule selon l'une des revendications précédentes, dans laquelle les groupes photoactivables sont choisis dans le groupe constitué des aryldiazirines et des benzophénones.

7. Macromolécule selon l'une des revendications précédentes, dans laquelle les groupes photoactivables sont choisis dans le groupe constitué la 4-(p-azidosalicylamido)butylamine, le N-hydroxysuccinimidyl-4-azidosalicylique acide, la p o-phénone-4-maléimide, le 4-benzylbenzoïque adique succimidyl ester, ou l' -azidophényl-isothiocyanate, le benzophénone-4-isothiocyanate, la benz 3-(trifluorométhyl)-3-(m-isothiocyanophényl) diazirine

8. Macromolécule selon l'une des revendications précédentes, dans laquelle le métal est choisi dans le groupe constitué de l'aluminium, le cuivre, l'or, le palladium, le platine et l'argent.

9. Surface captrice d'un biocapteur, qui comprend une macromolécule selon la revendication 2.

10. Microréseau, qui comprend une macromolécule selon la revendication 2.

11. Nanoparticule, nanoassemblage or microparticule comprenant une macromolécule selon la revendication 2.

12. Méthode pour préparer une macromolécule photolinker selon la revendication 1 qui comprend la dérivatisation d'un polysaccharide par substitution multiple avec des groupes photoactivables et des groupes contenant du soufre, et l'attachement du saccharide dérivatisé à un métal par des procédés de chimiesorption ou de formation de complexe métal-soufre.

13. Méthode pour préparer une macromolécule photolinker selon la revendication 2 qui comprend la soumission d'un mélange d' une macromolécule photolinker selon la revendication 1 et d'une biomolécule sous forme active à une photoréaction à une longueur d'onde d'au moins 320 nm, en l'absence de lumière incidente sous 320 nm.
